# EUROPEAN PATENT APPLICATION

(11) **EP 2 774 548 A2**
(43) Date of publication of application: **10.09.2014**
(21) Application number: 14157578.7
(22) Date of filing: 04.03.2014
(51) Int. Cl.: A61B 17/072

(54) **Surgical stapling device including adjustable fastener crimping**

(30) Priority: 05.03.2013 US 201313785138
(71) Applicant: Covidien LP, Mansfield, MA 02048 (US)
(72) Inventor: Mozdzierz, Patrick, Glastonbury, CT 06033 (US); Slisz, Kevin, Old Saybrook, CT Connecticut 06475 (US)
(74) Representative: Soames, Candida Jane

(57) **Abstract**

An end effector (100) includes an anvil member (120), a cartridge member(110), and a pivot plate (130, 230) connected to the anvil member and the cartridge member. The anvil member defines fastener forming pockets. The cartridge member defines fastener retaining slots. The cartridge member supports fasteners in the fastener retaining slots. The pivot plate is movable relative to the anvil member and the cartridge member to adjust a formation height of the fasteners upon a firing of the end effector. The pivot plate defines a channel (136, 232, 234) within a body portion of the pivot plate. At least one of the anvil member and the cartridge member includes a pin (115) extending therefrom. The channel is dimensioned to receive the pin. A movement of the pivot plate enables the pin to move along the channel and move at least one of the anvil member and the cartridge member relative to the other.

## Description

### TECHNICAL FIELD

This application relates to a surgical stapling device, and more particularly, to a surgical stapling device for forming surgical fasteners to body tissue at different crimp heights and/ or configurations.

### BACKGROUND

As medical and hospital costs continue to increase, surgeons are constantly striving to develop advanced surgical techniques. Advances in the surgical field are often related to the development of operative techniques which involve less invasive surgical procedures and reduce overall patient trauma. In this manner, the length of hospital stays can be significantly reduced, and, therefore, the hospital and medical costs can be reduced as well.

Generally, endoscopic surgery involves incising through body walls to access an underlying working space, for example, for viewing and/or operating on the ovaries, uterus, gall bladder, bowels, kidneys, appendix, etc. A surgical portal such as a cannula can be inserted through the incision to provide an access to the working space for elongate surgical instruments used to view and/or operate in the working space. As appreciated, surgical portals can be provided in various shapes and sizes to accommodate instruments of different sizes.

There are many common endoscopic surgical procedures, including arthroscopy, laparoscopy (pelviscopy), gastroentroscopy and laryngobronchoscopy, just to name a few. In order to address the specific needs of endoscopic surgical procedures, surgical stapling devices have been developed. Many of these surgical stapling devices are relatively expensive to manufacture, purchase and/or operate. To this end, there is a desire by manufactures and end users to develop effective surgical stapling devices that are relatively inexpensive to manufacture, purchase and/or operate.

Generally, surgical stapling devices include end effectors with a pair of jaw members that support loading units with fastener cartridges having a plurality of uniform fasteners. These jaw members define a tissue gap therebetween and cooperate to clamp, cut, and/or fasten tissue disposed within the tissue gap. However, all tissue is not uniform in thickness and, as a result, discrepancies in tissue thickness can compromise the effectiveness of the surgical fasteners if not properly secured within the tissue. Thus, during a fastening procedure, a fastener cartridge of a surgical stapling device may need to be replaced with another fastener cartridge that includes fasteners of a different size to properly accommodate tissue thickness variance. In some instances, the entire surgical stapling device may need to be replaced to be able to provide different sized fasteners. As can be appreciated, some end effectors/surgical stapling devices are larger than others, for example, to support larger fasteners. As such, these larger devices require larger incisions for enabling access to the underlying working space.

As such, a need exists to have a cost effective surgical stapling device that can be inserted through small surgical portals and can adjust one or both of the tissue gap and the fastener formation/crimp height without replacing the fastener cartridge to more effectively form surgical fasteners within different tissue thicknesses.

### SUMMARY

According to one aspect, the present disclosure relates to an end effector for a surgical stapling device including an anvil member, a cartridge member, and a pivot plate(s) connected to the anvil member and the cartridge member. The anvil member defines a plurality of fastener forming pockets in a tissue engaging surface thereof. The cartridge member defines a plurality of fastener retaining slots in a tissue engaging surface thereof. The cartridge member supports a plurality of fasteners in the fastener retaining slots.

The pivot plate is movable relative to the anvil member and the cartridge member to adjust a formation height of one or more of the plurality of fasteners upon a firing of the end effector. The pivot plate defines one or more channels within a body portion of the pivot plate. One or both of the anvil member and the cartridge member include a pin extending therefrom. The one or more channels are dimensioned to receive the pin. A movement of the pivot plate enables the pin to move along the one or more channels to move one or both of the anvil member and the cartridge member relative to the other of the anvil member and the cartridge member.

In embodiments, the pivot plate moves axially between a proximal position and a distal position. The one or more channels are disposed at an angle relative to a longitudinal axis defined through the end effector. The pivot plate can include a pair of arms extending from a backspan, wherein a first channel is defined through one of the pair of arms and a second channel is defined through the other arm of the pair of arms.

According to certain embodiments, the pivot plate is rotatable relative to the anvil member and the cartridge member. A rotation of the pivot plate moves the anvil member and the cartridge member relative to one another to adjust the formation height of one or more of the plurality of fasteners. The anvil member can include a first pin that extends therefrom and the cartridge member can include a second pin that extends therefrom. In embodiments, the pivot plate defines a first arcuate channel and a second arcuate channel. The first arcuate channel is dimensioned to receive the first pin. The second arcuate channel is dimensioned to receive the second pin. A rotation of the pivot plate enables the first pin to slide along the first arcuate channel and the second pin to slide along the second arcuate channel.

According to one aspect, a knife assembly for a surgical stapling device includes a base assembly, an adjusting assembly, and a knife.

The base assembly includes a body member and a support member. The adjusting assembly is movably supported on the support member and includes a linking member and a driving member. The driving member is rotatable to axially move the linking member.

The knife is pivotally coupled to the body member and pivotally coupled to the linking member. The knife is pivotable between a first orientation and a second orientation in response to a rotation of the driving member. The first orientation is different from the second orientation. The knife is positioned in coaxial alignment with a longitudinal axis defined by the knife assembly when disposed in the first orientation and axially offset from the longitudinal axis defined by the knife assembly when disposed in the second orientation. An axial movement of the linking member pivots a top portion of the knife proximally relative to a bottom portion of the knife from the first orientation to the second orientation. The top and bottom portions of the knife are longitudinally aligned in the first orientation and the top portion is disposed proximally of the bottom portion in the second orientation. The linking member moves between proximal and distal orientations. The knife defines a first height in the distal orientation and a second height in the proximal orientation. The first height and the second height are different.

In accordance with another aspect, a surgical stapling device includes an anvil member, a cartridge member, a pivot plate, and a knife assembly.

The anvil member defines a plurality of fastener forming pockets in a tissue engaging surface thereof. The cartridge member defines a plurality of fastener retaining slots in a tissue engaging surface thereof. The cartridge member supports a plurality of fasteners in the fastener retaining slots.

The pivot plate is connected to the anvil member and the cartridge member. The pivot plate is movable relative to the anvil member and the cartridge member to adjust a formation height of one or more of the plurality of fasteners upon a firing of the surgical stapling device. A movement of the pivot plate moves one or both of the anvil member and the cartridge member relative to the other of the anvil member and the cartridge member. The pivot plate defines one or more channels within a body portion of the pivot plate. One or both of the anvil member and the cartridge member includes a pin extending therefrom. The one or more channels are dimensioned to receive the pin. A movement of the pivot plate enables the pin to move along the one or more channels.

According to certain embodiments, the pivot plate moves axially between a proximal position and a distal position. In some embodiments, the pivot plate is rotatable relative to the anvil member and the cartridge member. A rotation of the pivot plate moves the anvil member and the cartridge member relative to one another to adjust the formation height of one or more of the plurality of fasteners. The anvil member includes a first pin that extends therefrom and the cartridge member includes a second pin that extends therefrom. The pivot plate defines a first arcuate channel and a second arcuate channel. The first arcuate channel is dimensioned to receive the first pin. The second arcuate channel is dimensioned to receive the second pin.

The knife assembly defines a longitudinal axis therethrough and is operatively coupled to one or both of the anvil member and the cartridge member. The knife assembly includes a base, an adjusting assembly, and a knife. The adjusting assembly is movably supported on the base and includes a linking member and a driving member. The driving member is rotatable to axially move the linking member. The linking member moves between proximal and distal orientations. The knife is pivotally coupled to the base and the linking member of the adjusting assembly. The knife is pivotable between a first orientation and a second orientation in response to a rotation of the driving member. The first orientation is different from the second orientation. The knife is positioned in coaxial alignment with a longitudinal axis defined by the knife assembly when disposed in the first orientation and axially offset from the longitudinal axis defined by the knife assembly when disposed in the second orientation. An axial movement of the linking member pivots a top portion of the knife proximally relative to a bottom portion of the knife from the first orientation to the second orientation. The top and bottom portions of the knife are longitudinally aligned in the first orientation and the top portion is disposed proximally of the bottom portion in the second orientation. The knife defines a first height in the distal orientation and a second height in the proximal orientation. The first height and the second height are different.

According to one aspect, the present disclosure is directed to a method for inserting an end effector into an opening having a first diameter, wherein the end effector includes a first jaw member and a second jaw member, and wherein outer surfaces of the first and second jaw members together have a second diameter. The method includes moving one or more pivot plates secured to the first and second jaw members to reduce a tissue gap defined between a tissue engaging surface of the first jaw member and a tissue engaging surface of the second jaw member, reducing the second diameter from a size larger than the first diameter to a size that is smaller than the first diameter by reducing the tissue gap, and inserting the end effector into the opening.

Other aspects, features, and advantages will be apparent from the description, drawings, and claims.

### BRIEF DESCRIPTION OF DRAWINGS

The accompanying drawings, which are incorporated in and constitute a part of this specification, illustrate embodiments of the disclosure and, together with a general description of the disclosure given above, and the detailed description given below, serve to explain the principles of the disclosure, wherein:
FIG. 1 is a perspective view of an endoscopic surgical stapling device according to the present disclosure;
FIG. 2 is an enlarged perspective cut-away view illustrating an embodiment of an end effector of the presently disclosed surgical stapling device during a fastener applying operation as fasteners are being sequentially fired;
FIG. 3 is an enlarged bottom perspective view of a distal portion of an anvil member of the end effector of FIG. 2;
FIG. 4A is a side view, with parts separated, of the end effector of FIG. 2;
FIG. 4B is a side view of an embodiment of a pivot plate of the end effector of FIG. 2;
FIGS. 5 and 6 are side views of the end effector of FIG. 2 illustrating different configurations of the end effector;
FIGS. 7 and 8 are side views of proximal portions of another embodiment of an end effector in accordance with the present disclosure, the end effector being illustrated in a different configuration in each view;
FIGS. 9 and 10 are side views of an embodiment of a knife assembly in accordance with the present disclosure, the knife assembly being illustrated in a different configuration in each view;
FIG. 11 is a graphical illustration of a relationship between tissue gap height and amount of fastener crimp provided by the presently disclosed end effector; and
FIGS. 12 and 13 are side views of formed fasteners, each fastener illustrating a different amount of fastener crimp.

### DETAILED DESCRIPTION

As used herein, the term "clinician" refers to a doctor, a nurse, or any other care provider and may include support personnel. Particular embodiments of the present disclosure will be described herein with reference to the accompanying drawings. As shown in the drawings and as described throughout the following description, and as is traditional when referring to relative positioning on an object, the term "proximal" refers to the end of the device that is closer to the clinician and the term "distal" refers to the end of the device that is farther from the clinician. In the following description, well-known functions or constructions are not described in detail to avoid obscuring the present disclosure in unnecessary detail.

In general, linear stapling devices, including open and endoscopic devices, can have two elongated jaw members which are respectively used to capture or clamp tissue. Typically, one of the jaw members carries a fastener cartridge which houses a plurality of fasteners arranged in at least two lateral rows of fastener retaining slots defined with the fastener cartridge while the other jaw member has an anvil member that defines a surface with a plurality of fastener forming pockets for forming the fastener legs as the fasteners are driven from the fastener cartridge. Generally, the fastening operation is effected by cam bars or a sled that travel longitudinally through the fastener cartridge, with the cam bars or sled acting upon pusher members to sequentially eject the fasteners from the fastener cartridge. A knife can travel between the fastener rows to longitudinally cut and/or open the fastened tissue between the rows of fasteners. Such an instrument is disclosed, for example, in U.S. Pat. No. 6,202,914, the entire contents of which are incorporated herein by reference.

Some stapling devices apply a double row of staples on each side of the incision. This is accomplished by providing a disposable loading unit in which a cam member moves through an elongate guide path between two sets of staggered fastener carrying grooves. Fastener drive members are located within the grooves and are positioned in such a manner so as to be contacted by the longitudinally moving cam member to effect ejection of the fasteners from the fastener cartridge of the disposable loading unit. An example of such a stapling device is disclosed in U.S. Pat. Nos. 5,065,929, the entire contents of which are incorporated herein by reference.

Some of the instruments described above were designed for use in conventional surgical procedures wherein surgeons have direct manual access to the operative site. However, in endoscopic or laparoscopic procedures, surgery is performed through a small incision or through a narrow cannula inserted through small entrance wounds in the skin. In order to address the specific needs of endoscopic and/or laparoscopic surgical procedures, endoscopic surgical stapling devices have been developed and are disclosed in, for example, U.S. Pat. No. 5,865,361, the entire contents of which are incorporated herein by reference.

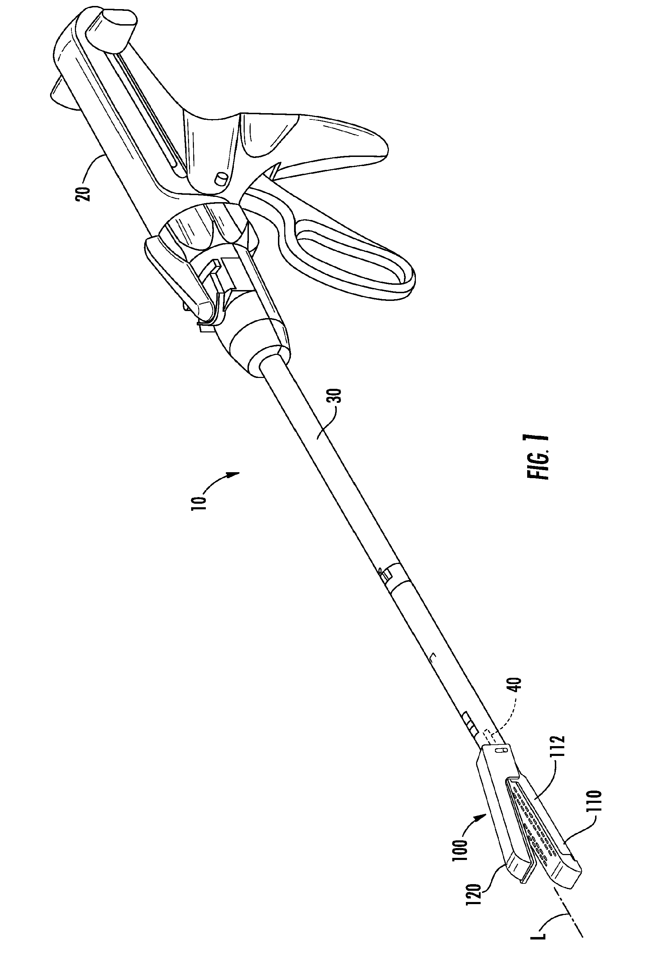

Referring now to the drawings, in which like reference numerals identify identical or substantially similar parts throughout the several views, FIG. 1 illustrates a surgical stapling device 10 in accordance with the present disclosure that may be arranged for use with minimally invasive or open stapling procedures. The surgical stapling device 10 includes a housing 20 and an elongate member 30 that extends distally from the housing 20. A distal end of the elongate member 30 supports a proximal end of an end effector 100. The end effector 100 defines a longitudinal axis "L" between the proximal and distal ends of the end effector 100 and includes a cartridge member 110 that is dimensioned to selectively receive a staple cartridge 112 and an anvil member 120. The cartridge member 110 and the anvil member 120 are pivotably coupled at proximal ends thereof and may each support a surgical buttress (not shown). The staple cartridge 112 houses staples and/or other surgical fasteners other than staples. The cartridge member 110 and the staple cartridge 112 may be selectively replaceable, either individually or collectively.

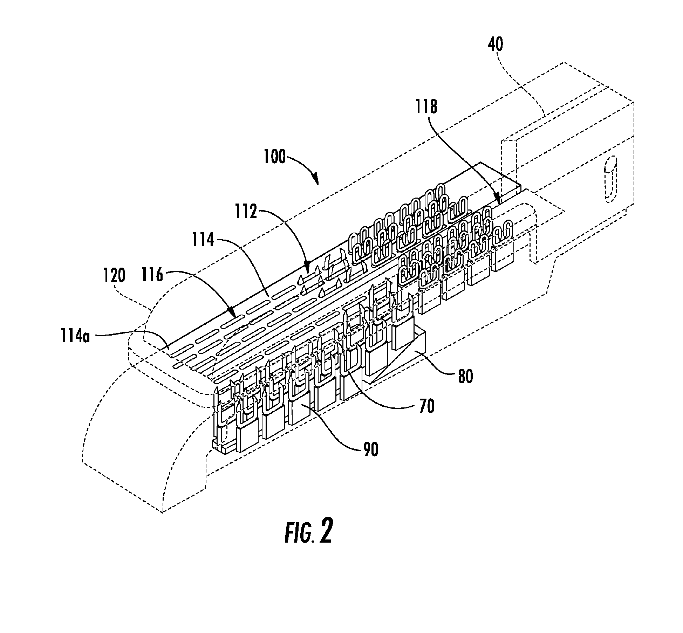

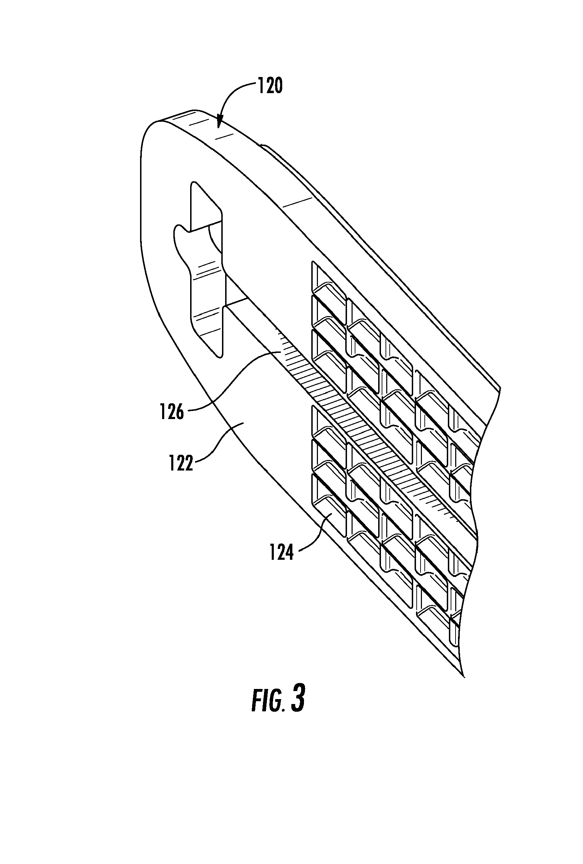

With reference to FIGS. 2 and 3, the staple cartridge 112 includes a cartridge housing 114 including a tissue contacting surface 114a having a plurality of rows of fastener retaining slots 116 formed therein that house a plurality of fasteners 70. The staple cartridge 112 defines a cartridge knife channel 118 between proximal and distal ends of the staple cartridge 112 that is adapted to receive a knife 40 that translates distally through the cartridge knife channel 118 to cut tissue clamped between the cartridge assembly 110 and the anvil assembly 120. The plurality of fasteners 70 may be sequentially formed in fastener forming pockets 124 defined in a tissue contacting surface 122 of the anvil member 120 upon a distal advancement of a cam bar and/or a sled 80 supported in the cartridge housing 114 into a plurality of fastener pusher members 90 supported in the cartridge housing 114. The anvil member 120 defines an anvil knife channel 126 between proximal and distal ends of the anvil member 120 that receives the knife 40 as the knife 40 translates distally through the cartridge knife slot 118.

In any of the embodiments disclosed herein, the cartridge assembly can be a removable and replaceable unit, the entire end effector 100 may be removable and replaceable, or both. Desirably the surgical instrument includes one or more parts or assemblies that can be reused at least during the same surgical procedure. Furthermore, the handle assembly can be manually operated or contain or be attached to a motor and power source. Electrical and other power sources are contemplated.

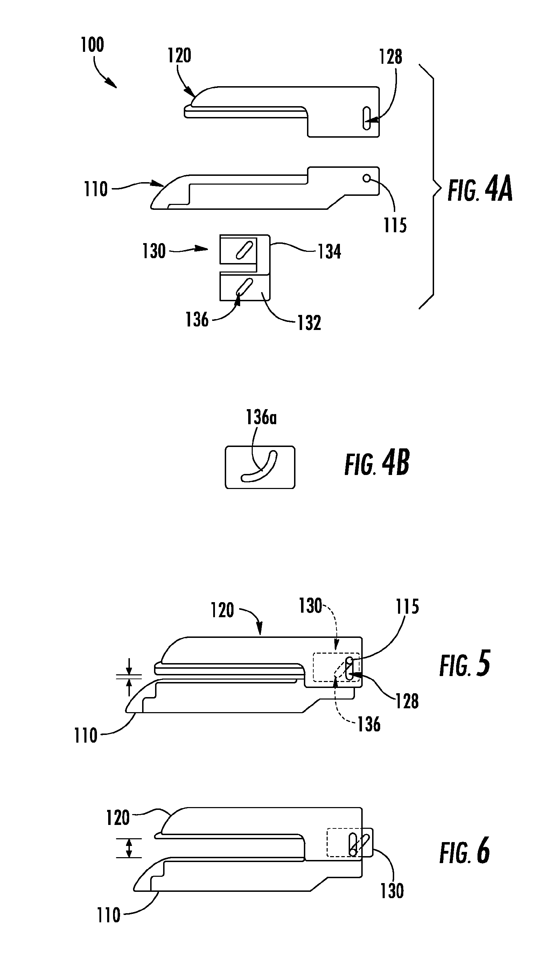

As shown in FIGS. 4A, 5, and 6, a pivot plate 130 (or a plurality of pivot plates 130) is connected to the anvil member 120 and the cartridge member 110. The pivot plate 130 is movable relative to the anvil member 120 and the cartridge member 110 to adjust a formation/crimp height of one or more of the plurality of fasteners 70 upon a firing of the end effector 100.

The pivot plate 130 has a generally U-shaped body and includes a pair of arms 132 supported on opposed ends of a backspan 134. The arms 132 are dimensioned to be secured on laterally opposite sides of the end effector. In embodiments, the U-shaped body is integrally formed. Each arm 132 defines a channel 136 therethrough, which as shown in FIG. 4A can have linear slope. Each channel 136 is disposed at an angle relative to the longitudinal axis "L" defined by the end effector 100. Notably, the angle may be any suitable angle including acute and obtuse angles. As shown in FIG. 4B, each arm 132 can define a channel 136a that has a curved or stepped slope to provide a changing sensitivity and enable the cartridge and anvil members 110, 120 to be approximated and/or unapproximated at different speeds as one or more pins 115 of one or both of the anvil and/or cartridge members 110, 120, slide through the channel 136a to effectuate movement of one or both of the anvil and/or cartridge members 110, 120 as will be described in greater detail below.

One or both of the anvil member 120 and the cartridge member 110 defines one or more openings 128 and one or both of the anvil member 120 and the cartridge member 110 includes one or more pins 115 extending from one or more side surfaces thereof. Each opening 128 and each channel 136, 136a is dimensioned to receive one or more of the pins 115 so that the pivot plate 130, the anvil member 120 and the cartridge member 110 are movably connected. For example, the anvil member 120 can define an opening within each side surface thereof and the cartridge member 110 can include a pin 115 extending from each side surface thereof which is received within channels 136, 136a of the pivot plate 130 and the openings 128 of the anvil member 120. In this regard, there is relative movement between the anvil member 120 and the cartridge member 110 as the pins 115 simultaneously slide within the openings 128 of the anvil member 120 and the channels 136, 136a of the pivot plate 130.

An axial movement of the pivot plate 130 via any suitable drive assembly (not shown) enables the cartridge member 110 and the anvil member 120 to move relative to one another in a direction orthogonal to the longitudinal axis "L" defined by the end effector "L" when the one or more pins 115 are received within the one or more openings 128 and the one or more channels 136, 136a. In particular, the one or more pins 115 slide within the one or more openings 128 and the one or more channels 136, 136a as the pivot plate 130 moves between proximal and distal positions. As appreciated, the channels 136, 136a, pins 115, and openings 128 can be arranged in any suitable configuration for facilitating movement of one or both of the cartridge member 110 and the anvil member 120 relative to one another to change a height of a tissue gap defined between the opposing tissue contacting surfaces of the anvil member 120 and the cartridge member 110. Notably, an adjustment of the tissue gap height adjusts a formation/crimp height of one or more of the plurality of fasteners 70 upon a firing of the end effector 100.

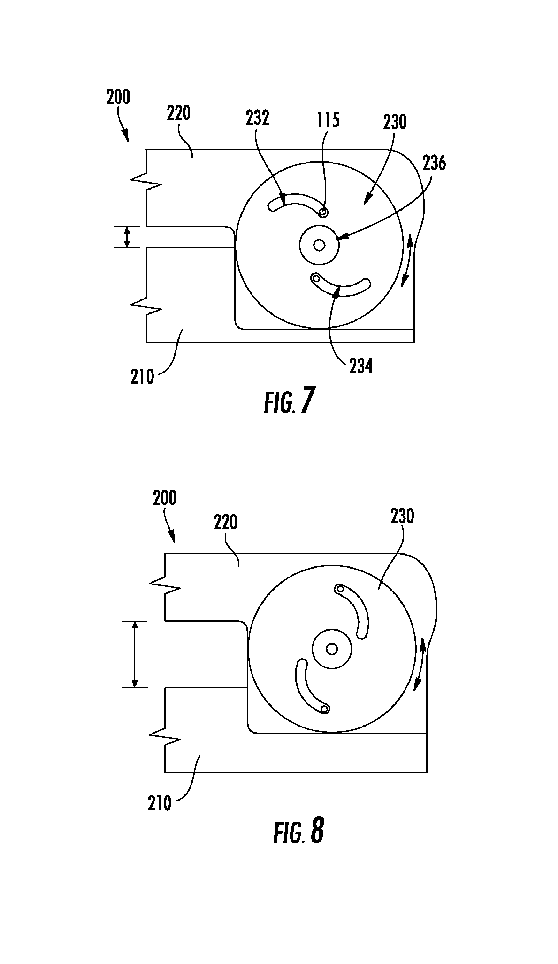

Turning now to FIGS. 7 and 8, another embodiment of the presently disclosed end effector is generally referred to as end effector 200. End effector 200 is substantially similar to end effector 100 and will only be described herein to the extent necessary to describe the differences in construction and operation. End effector 200 includes a cartridge member 210, an anvil member 220, and a pivot plate 230 (or a plurality of pivot plates 230) that is rotatably mounted to one or both of the cartridge member 210 and the anvil member 220 via a supporting member 236. Pivot plate 230 has a circular body which may be any suitable rounded arrangement including elliptical, semi-circular, crescent, or the like. Each of the cartridge member 210 and the anvil member 220 includes pin 115 extending from a side surface thereof. The pivot plate 230 has a substantially circular body that defines a first arcuate channel 232 and a second arcuate channel 234 which are dimensioned to receive the pins 115 extending from the cartridge member 210 and the anvil member 220. The pins 115 slide within the first and second arcuate channels 232, 234 to adjust the height of the tissue gap between the tissue engaging surfaces of the cartridge member 210 and the anvil member 220 upon a rotational movement of the pivot plate 230.

Any suitable drive assembly (not shown) is operatively coupled to pivot plate 130 to enable the cartridge member 210 and the anvil member 220 to move relative to one another in a direction orthogonal to the longitudinal axis "L" defined by the end effector "L" when the pins 115 are received within the arcuate channels 232, 234. In particular, the pins 115 slide along the arcuate channels 232, 234 as the pivot plate 230 rotates. As appreciated, the arcuate channels 232, 234 and pins 115 can be arranged in any suitable configuration for facilitating movement of one or both of the cartridge member 210 and the anvil member 220 relative to one another to change a height of a tissue gap defined between the opposing tissue contacting surfaces of the anvil member 220 and the cartridge member 210. As described above, an adjustment of the tissue gap height between the tissue engaging surfaces of the anvil and cartridge members adjusts a formation/crimp height of one or more of the plurality of fasteners 70 upon a firing of the end effector 200.

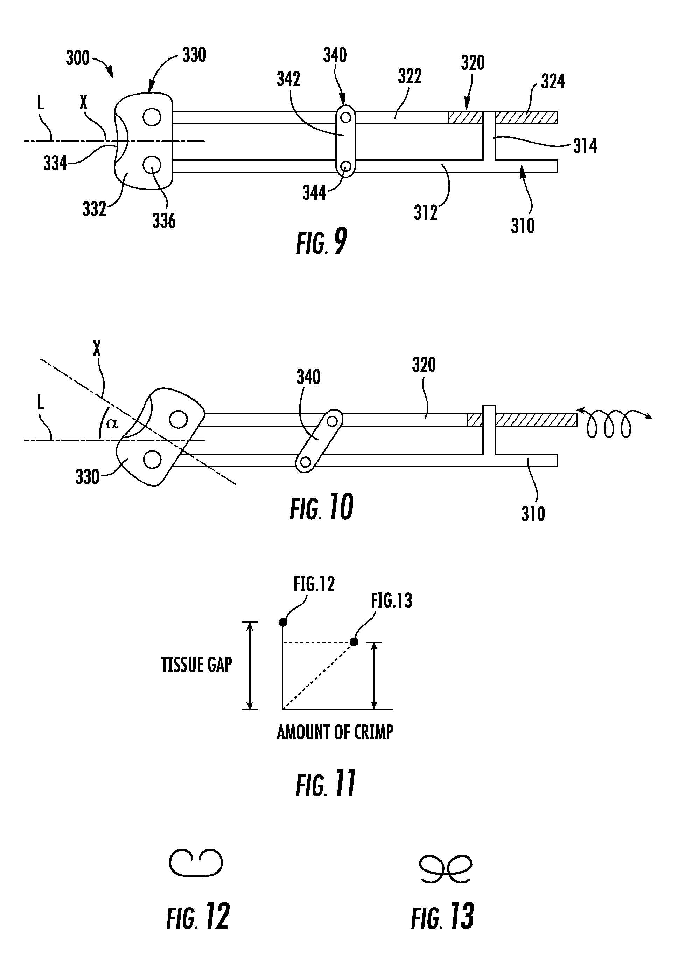

With reference to FIGS. 9-13, embodiments of the presently disclosed surgical stapling device include a knife assembly generally referred to as knife assembly 300. Knife assembly 300 includes a base assembly 310, an adjusting assembly 320, a knife 330, and connecting member 340. The base assembly 310 includes a body member 312 and a support member 314 that extends from the body member 312 at a proximal end portion of the body member 312. The adjusting assembly 320 includes a linking member 322 and driving member 324. The driving member 324 is threadably engaged with the support member 314. A distal end portion of the driving member 324 is connected to a proximal end portion of the linking member 322. The knife 330 includes a knife body 332 that supports a knife blade 334 on a distal end portion of the knife body 332. A proximal end portion of the knife body 332 is secured via pins 336 (or any other suitable mechanical connection) to a distal end of the linking member 322 of the adjusting assembly 320 and a distal end of the body member 312 of the base assembly 310. The connecting member 340 includes a connecting member body 342 that is secured between the body member 312 and the linking member 322. One end portion of the connecting member 340 is secured to the body member 312 by one pin 344 (or any other suitable mechanical connection) and the other end portion of the connecting member 340 is secured to the linking member 322 by another pin 344 (or any other suitable mechanical connection).

In embodiments, the knife body 332 and the linking member 322 extend laterally outwardly in an I-beam type configuration. The laterally outwardly extending portions (e.g., flanges) of the knife body 332 and/or linking member 322 are slidable through channels defined respectively within the anvil and cartridge members to enable the anvil and cartridge members to remain disposed in a fixed position relative to one another when the knife assembly 300 (or portions thereof) is distally advanced between the anvil and cartridge members.

A proximal end portion of the driving member 324 is coupled to a drive assembly (not shown) that rotates the driving member 324 relative to the support member 314 to axially move the linking member 322. For example, a threaded arrangement between member 324 and member 322 can be used. Axial movement of the linking member 322 pivots the knife 330 about the pins 336 to move the knife blade 334 between a first orientation and a second orientation, which includes one or more intermediate orientations between the first and second orientations. In embodiments, a sliding knob (not shown) is directly connected to the linking member 322 and/or the body member 312 to effectuate movement of the knife 330 upon a sliding movement of the sliding knob. In one of the orientations of the knife 330, a knife axis "X" defined by the knife 330, which extends centrally through the knife 330 between a proximal portion and a distal portion of the knife 330, is coaxial with the longitudinal axis "L" of the end effector. In this orientation, a top portion and a bottom portion of the knife 330 are longitudinally aligned such that the tissue gap height is at a maximum. In another orientation of the knife 330, the knife axis "X" is disposed at an angle α relative to the longitudinal axis "L" such that the top portion of the knife 330 is disposed proximally of the bottom portion of the knife 330. Angle α may be any suitable acute angle relative to the longitudinal axis "L." Notably, the knife 330 is positioned at angle α to enable the knife assembly 300 to advance through the end effector when the height of the tissue gap is less than the maximum tissue gap height. In this manner, as illustrated in FIGS. 11-13, there is a direct relationship between the tissue gap height (and thus, formation/crimp height of the fasteners 70) and the angle α.

The knife 330 is positioned in coaxial alignment with the longitudinal axis "L" of the end effector when disposed in the first orientation and axially offset from the longitudinal axis "L" when disposed in the second orientation. An axial movement of the linking member 322 pivots a top portion of the knife 330 proximally relative to a bottom portion of the knife 330 from the first orientation to the second orientation. The top and bottom portions of the knife 330 are longitudinally aligned in the first orientation and the top portion is disposed proximally of the bottom portion in the second orientation. In particular, when the knife 330 is in the first orientation, a pivot point of the top portion of the knife 330 is longitudinally aligned with a pivot point of the bottom portion of the knife 330 relative to the longitudinal axis "L." In the second orientation, the pivot point of the top portion of the knife 330 is disposed proximally of the pivot point of the bottom portion of the knife 330.

As discussed above, a surgical portal used to provide access to an underlying working space defines an opening to permit the passage of surgical instruments such as surgical stapling device 10 therethrough. However, to minimize the size of a surgical incision, it is desirable to use the smallest practical surgical portal to effectively conduct the procedure. In accordance with the present disclosure, a clinician can utilize smaller surgical portals to access underlying working spaces by virtue of the adjustable end effector of the presently disclosed surgical stapling devices 10. For example, the pivot plate(s) 130, 230 secured to the anvil member 120, 220 and the cartridge member 110, 210 can be moved to enlarge and/or reduce the tissue gap defined between the tissue engaging surface of the anvil member 120, 220 and the tissue engaging surface of the cartridge member 110, 210. For example, the anvil member 120, 220 and the cartridge member 110, 210 can be moved into contact such that there is zero tissue gap between the cartridge member 110, 210 and the anvil member 120, 220. In this regard, the end effector maintains its smallest profile. Notably, when the anvil member 120, 220 and the cartridge member 110, 210 are moved (via the pivot plate(s) 130, 230) to a position in which they are spaced farthest apart, the end effector maintains its largest profile. Upon a movement of the pivot plate(s) 130, 230 to reduce the tissue gap, the collective profile of the anvil and cartridge members of the end effector can be reduced from a size that is larger than the diameter of the opening of the surgical portal to a size that is smaller than the diameter of the opening of the surgical portal. When the profile of the end effector is reduced to a size smaller than the opening of the surgical portal, the end effector can be advanced through the opening.

After inserting the surgical stapling device 10 through the surgical portal and into the working space, the cartridge member 110, 210 and the anvil member 120, 220 are clamped against tissue of a patient. The pivot plate 130, 230 may be moved to adjust the tissue gap height for adjusting the formation/crimp height of the fasteners 70 upon firing. The knife assembly 300 may also be pivoted to adjust the knife height until the knife 330 is positioned in one of the first and second orientations commensurate with the tissue gap height so that the knife height and the tissue gap height are compatible to enable the knife assembly 300 to axially advance through the end effector. The surgical stapling device 10 is fired to deploy the fasteners 70 through the fastener retaining slots 116. Upon firing, the fasteners 70 pass through the fastener retaining slots 116 so that the legs of the fasteners 70 penetrate through the tissue. The fasteners 70 are then formed against the fastener forming pockets 124 of the anvil member 120, 220 thereby affixing the fasteners 70 to the tissue. Concomitantly therewith, the knife assembly 300 translatably disposed within the end effector 100, cuts through the tissue clamped between the cartridge member 110, 210 and the anvil member 120, 220 as the knife assembly 300 is distally translated through the end effector along the knife channels 118, 126 by a drive assembly (not shown).

Persons skilled in the art will understand that the structures and methods specifically described herein and illustrated in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. For example, the staples or surgical fasteners can be provided with an initial size or configuration that are all the same, or can vary, such as providing a cartridge assembly with different sized staples. In addition, the cartridge assembly can have a tissue engaging face that stepped, angled, etc. Additionally, it is envisioned that the elements and features illustrated or described in connection with one exemplary embodiment may be combined with the elements and features of another without departing from the scope of the present disclosure, and that such modifications and variations are also intended to be included within the scope of the present disclosure. Indeed, any combination of any of the presently disclosed elements and features is within the scope of the present disclosure. Accordingly, the subject matter of the present disclosure is not to be limited by what has been particularly shown and described.

The invention may be described by reference to the following numbered paragraphs:-
1. An end effector for a surgical stapling device, the end effector comprising:
   an anvil member defining a plurality of fastener forming pockets in a tissue engaging surface thereof;
   a cartridge member defining a plurality of fastener retaining slots in a tissue engaging surface thereof, the cartridge member supporting a plurality of fasteners in the fastener retaining slots; and
   at least one pivot plate connected to the anvil member and the cartridge member, the at least one pivot plate having one of a U-shaped body or a circular body, the at least one pivot plate being movable relative to the anvil member and the cartridge member to adjust a formation height of one or more of the plurality of fasteners upon a firing of the end effector, the at least one pivot plate defining at least one channel within the at least one pivot plate, at least one of the anvil member and the cartridge member including a pin extending therefrom, the at least one channel being dimensioned to receive the pin, wherein a movement of the at least one pivot plate enables the pin to move along the at least one channel to move at least one of the anvil member and the cartridge member relative to the other of the anvil member and the cartridge member.
2. The end effector of paragraph 1, wherein the at least one pivot plate moves axially between a proximal position and a distal position.
3. The end effector of paragraph 2, wherein the at least one channel is disposed at an angle relative to a longitudinal axis defined through the end effector.
4. The end effector of paragraph 3, wherein a first channel is defined through one of the pair of arms and a second channel is defined through the other arm of the pair of arms.
5. The end effector of paragraph 1, wherein the at least one pivot plate is rotatable relative to the anvil member and the cartridge member, a rotation of the at least one pivot plate moving the anvil member and the cartridge member relative to one another to adjust the formation height of one or more of the plurality of fasteners.
6. The end effector of paragraph 5, wherein the anvil member includes a first pin that extends therefrom and the cartridge member includes a second pin that extends therefrom, the at least one pivot plate defining a first arcuate channel and a second arcuate channel, the first arcuate channel dimensioned to receive the first pin, the second arcuate channel dimensioned to receive the second pin.
7. The end effector of paragraph 6, wherein a rotation of the at least one pivot plate enables the first pin to slide along the first arcuate channel and the second pin to slide along the second arcuate channel.
8. A knife assembly for a surgical stapling device, the knife assembly comprising:
   a base assembly including a body member and a support member;
   an adjusting assembly movably supported on the support member and including a linking member and a driving member, the driving member being rotatable to axially move the linking member; and
   a knife pivotally coupled to the body member and pivotally coupled to the linking member, the knife being pivotable between a first orientation and a second orientation in response to a rotation of the driving member, the first orientation being different from the second orientation.
9. The knife assembly of paragraph 8, wherein the knife is positioned in coaxial alignment with a longitudinal axis defined by the knife assembly when disposed in the first orientation and axially offset from the longitudinal axis defined by the knife assembly when disposed in the second orientation.
10. The knife assembly of paragraph 8, wherein an axial movement of the linking member pivots a top portion of the knife proximally relative to a bottom portion of the knife from the first orientation to the second orientation, the top and bottom portions of the knife being longitudinally aligned in the first orientation and the top portion being disposed proximally of the bottom portion in the second orientation.
11. The knife assembly of paragraph 8, wherein the linking member moves between proximal and distal orientations, the knife defining a first height in the distal orientation and a second height in the proximal orientation, the first height and the second height being different.
12. A surgical stapling device, comprising:
   an anvil member defining a plurality of fastener forming pockets in a tissue engaging surface thereof;
   a cartridge member defining a plurality of fastener retaining slots in a tissue engaging surface thereof, the cartridge member supporting a plurality of fasteners in the fastener retaining slots;
   a pivot plate connected to the anvil member and the cartridge member, the pivot plate being movable relative to the anvil member and the cartridge member to adjust a formation height of one or more of the plurality of fasteners upon a firing of the surgical stapling device, wherein a movement of the pivot plate moves at least one of the anvil member and the cartridge member relative to the other of the anvil member and the cartridge member;
   a knife assembly defining a longitudinal axis therethrough and being operatively coupled to at least one of the anvil member and the cartridge member, the knife assembly including:
      a base;
      an adjusting assembly movably supported on the base and including a linking member and a driving member, the driving member being rotatable to axially move the linking member; and
      a knife pivotally coupled to the base and the linking member of the adjusting assembly, the knife being pivotable between a first orientation and a second orientation in response to a rotation of the driving member, the first orientation being different from the second orientation.
13. The surgical stapling device of paragraph 12, wherein the pivot plate defines at least one channel within a body portion of the pivot plate, and wherein at least one of the anvil member and the cartridge member includes a pin extending therefrom, the at least one channel being dimensioned to receive the pin.
14. The surgical stapling device of paragraph 13, wherein a movement of the pivot plate enables the pin to move along the at least one channel.
15. The surgical stapling device of paragraph 12, wherein the pivot plate moves axially between a proximal position and a distal position.
16. The surgical stapling device of paragraph 12, wherein the pivot plate is rotatable relative to the anvil member and the cartridge member, and wherein a rotation of the pivot plate moves the anvil member and the cartridge member relative to one another to adjust the formation height of one or more of the plurality of fasteners.
17. The surgical stapling device of paragraph 16, wherein the anvil member includes a first pin that extends therefrom and the cartridge member includes a second pin that extends therefrom, the pivot plate defining a first arcuate channel and a second arcuate channel, the first arcuate channel dimensioned to receive the first pin, the second arcuate channel dimensioned to receive the second pin.
18. The surgical stapling device of paragraph 12, wherein the knife is positioned in coaxial alignment with a longitudinal axis defined by the knife assembly when disposed in the first orientation and axially offset from the longitudinal axis defined by the knife assembly when disposed in the second orientation.
19. The surgical stapling device of paragraph 12, wherein an axial movement of the linking member pivots a top portion of the knife proximally relative to a bottom portion of the knife from the first orientation to the second orientation, the top and bottom portions of the knife being longitudinally aligned in the first orientation and the top portion being disposed proximally of the bottom portion in the second orientation.
20. The surgical stapling device of paragraph 12, wherein the linking member moves between proximal and distal orientations, the knife defining a first height in the distal orientation and a second height in the proximal orientation, the first height and the second height being different.
21. A method for inserting an end effector into an opening having a first diameter, wherein the end effector includes a first jaw member and a second jaw member, and wherein outer surfaces of the first and second jaw members together have a second diameter, the method comprising:
   moving at least one pivot plate secured to the first and second jaw members to reduce a tissue gap defined between a tissue engaging surface of the first jaw member and a tissue engaging surface of the second jaw member;
   reducing the second diameter from a size larger than the first diameter to a size that is smaller than the first diameter by reducing the tissue gap; and
   inserting the end effector into the opening.

## Claims

1. An end effector for a surgical stapling device, the end effector comprising:
an anvil member defining a plurality of fastener forming pockets in a tissue engaging surface thereof;
a cartridge member defining a plurality of fastener retaining slots in a tissue engaging surface thereof, the cartridge member supporting a plurality of fasteners in the fastener retaining slots; and
at least one pivot plate connected to the anvil member and the cartridge member, the at least one pivot plate having one of a U-shaped body or a circular body, the at least one pivot plate being movable relative to the anvil member and the cartridge member to adjust a formation height of one or more of the plurality of fasteners upon a firing of the end effector, the at least one pivot plate defining at least one channel within the at least one pivot plate, at least one of the anvil member and the cartridge member including a pin extending therefrom, the at least one channel being dimensioned to receive the pin, wherein a movement of the at least one pivot plate enables the pin to move along the at least one channel to move at least one of the anvil member and the cartridge member relative to the other of the anvil member and the cartridge member.

2. The end effector of claim 1, wherein the at least one pivot plate moves axially between a proximal position and a distal position, wherein the at least one channel is disposed at an angle relative to a longitudinal axis defined through the end effector, preferably wherein a first channel is defined through one of the pair of arms and a second channel is defined through the other arm of the pair of arms.

3. The end effector of claim 1, wherein the at least one pivot plate is rotatable relative to the anvil member and the cartridge member, a rotation of the at least one pivot plate moving the anvil member and the cartridge member relative to one another to adjust the formation height of one or more of the plurality of fasteners, preferably wherein the anvil member includes a first pin that extends therefrom and the cartridge member includes a second pin that extends therefrom, the at least one pivot plate defining a first arcuate channel and a second arcuate channel, the first arcuate channel dimensioned to receive the first pin, the second arcuate channel dimensioned to receive the second pin.

4. The end effector of claim 3, wherein a rotation of the at least one pivot plate enables the first pin to slide along the first arcuate channel and the second pin to slide along the second arcuate channel.

5. A knife assembly for a surgical stapling device, the knife assembly comprising:
a base assembly including a body member and a support member;
an adjusting assembly movably supported on the support member and including a linking member and a driving member, the driving member being rotatable to axially move the linking member; and
a knife pivotally coupled to the body member and pivotally coupled to the linking member, the knife being pivotable between a first orientation and a second orientation in response to a rotation of the driving member, the first orientation being different from the second orientation.

6. The knife assembly of claim 5, wherein the knife is positioned in coaxial alignment with a longitudinal axis defined by the knife assembly when disposed in the first orientation and axially offset from the longitudinal axis defined by the knife assembly when disposed in the second orientation.

7. The knife assembly of claim 5 or claim 6, wherein an axial movement of the linking member pivots a top portion of the knife proximally relative to a bottom portion of the knife from the first orientation to the second orientation, the top and bottom portions of the knife being longitudinally aligned in the first orientation and the top portion being disposed proximally of the bottom portion in the second orientation.

8. The knife assembly of any of claims 5 to 7, wherein the linking member moves between proximal and distal orientations, the knife defining a first height in the distal orientation and a second height in the proximal orientation, the first height and the second height being different.

9. A surgical stapling device, comprising:
an anvil member defining a plurality of fastener forming pockets in a tissue engaging surface thereof;
a cartridge member defining a plurality of fastener retaining slots in a tissue engaging surface thereof, the cartridge member supporting a plurality of fasteners in the fastener retaining slots;
a pivot plate connected to the anvil member and the cartridge member, the pivot plate being movable relative to the anvil member and the cartridge member to adjust a formation height of one or more of the plurality of fasteners upon a firing of the surgical stapling device, wherein a movement of the pivot plate moves at least one of the anvil member and the cartridge member relative to the other of the anvil member and the cartridge member;
a knife assembly defining a longitudinal axis therethrough and being operatively coupled to at least one of the anvil member and the cartridge member, the knife assembly including:
a base;
an adjusting assembly movably supported on the base and including a linking member and a driving member, the driving member being rotatable to axially move the linking member; and
a knife pivotally coupled to the base and the linking member of the adjusting assembly, the knife being pivotable between a first orientation and a second orientation in response to a rotation of the driving member, the first orientation being different from the second orientation.

10. The surgical stapling device of claim 9, wherein the pivot plate defines at least one channel within a body portion of the pivot plate, and wherein at least one of the anvil member and the cartridge member includes a pin extending therefrom, the at least one channel being dimensioned to receive the pin, preferably wherein a movement of the pivot plate enables the pin to move along the at least one channel.

11. The surgical stapling device of claim 9 or claim 10, wherein the pivot plate moves axially between a proximal position and a distal position.

12. The surgical stapling device of any of claims 9 to 11, wherein the pivot plate is rotatable relative to the anvil member and the cartridge member, and wherein a rotation of the pivot plate moves the anvil member and the cartridge member relative to one another to adjust the formation height of one or more of the plurality of fasteners, preferably wherein the anvil member includes a first pin that extends therefrom and the cartridge member includes a second pin that extends therefrom, the pivot plate defining a first arcuate channel and a second arcuate channel, the first arcuate channel dimensioned to receive the first pin, the second arcuate channel dimensioned to receive the second pin.

13. The surgical stapling device of any of claims 9 to 12, wherein the knife is positioned in coaxial alignment with a longitudinal axis defined by the knife assembly when disposed in the first orientation and axially offset from the longitudinal axis defined by the knife assembly when disposed in the second orientation.

14. The surgical stapling device of any of claims 9 to 13, wherein an axial movement of the linking member pivots a top portion of the knife proximally relative to a bottom portion of the knife from the first orientation to the second orientation, the top and bottom portions of the knife being longitudinally aligned in the first orientation and the top portion being disposed proximally of the bottom portion in the second orientation, and/or wherein the linking member moves between proximal and distal orientations, the knife defining a first height in the distal orientation and a second height in the proximal orientation, the first height and the second height being different.

15. A method for inserting an end effector into an opening having a first diameter, wherein the end effector includes a first jaw member and a second jaw member, and wherein outer surfaces of the first and second jaw members together have a second diameter, the method comprising:
moving at least one pivot plate secured to the first and second jaw members to reduce a tissue gap defined between a tissue engaging surface of the first jaw member and a tissue engaging surface of the second jaw member;
reducing the second diameter from a size larger than the first diameter to a size that is smaller than the first diameter by reducing the tissue gap; and
inserting the end effector into the opening.
